(19) Europäisches Patentamt
European Patent Office
Office européen des brevets

(11) Veröffentlichungsnummer: **0 022 192**
**B1**

(12) # EUROPÄISCHE PATENTSCHRIFT

(45) Veröffentlichungstag der Patentschrift: **18.05.83**

(51) Int. Cl.³: **C 07 D 211/46,** C 07 H 15/12, A 61 K 31/445

(21) Anmeldenummer: **80103367.1**

(22) Anmeldetag: **18.06.80**

(54) **1-Alkadien-2,4-yl-2-hydroxymethyl-3,4,5-trihydroxy-piperidine, Verfahren zu ihrer Herstellung und ihre Verwendung als Arzneimittel.**

(30) Priorität: **27.06.79 DE 2925943**

(43) Veröffentlichungstag der Anmeldung: **14.01.81 Patentblatt 81/2**

(45) Bekanntmachung des Hinweises auf die Patenterteilung: **18.05.83 Patentblatt 83/20**

(84) Benannte Vertragsstaaten: **AT BE CH DE FR GB IT LI SE**

(56) Entgegenhaltungen:
**EP-A-0 000 947**
**EP-A-0 007 040**
**DE-A-2 824 781**
**DE-A-2 909 646**
**GB-A-2 020 278**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Junge, Bodo, Dr., Wilkhausstrasse 123, D-5600 Wuppertal 2 (DE)**
Erfinder: **Müller, Lutz, Dr., Kronprinzenallee 111, D-5600 Wuppertal 1 (DE)**
Erfinder: **Sitt, Rüdiger, Dr., Claudiusweg 9, D-5600 Wuppertal 1 (DE)**
Erfinder: **Thomas, Günter, Dr., Claudiusweg 9, D-5600 Wuppertal 1 (DE)**
Erfinder: **Krause, Hans Peter, Dr., Wilkhausstrasse 107, D-5600 Wuppertal 2 (DE)**
Erfinder: **Puls, Walter, Dr., In den Birken 75, D-5600 Wuppertal 1 (DE)**

**EP 0 022 192 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1-Alkadien-2,4-yl-2-hydroxymethyl-3,4,5-trihydroxypiperidine, Verfahren zu ihrer Herstellung und ihre Verwendung als Arzneimittel

Die vorliegende Erfindung betrifft neue Derivate von 2-Hydroxymethyl-3,4,5-trihydroxypiperidin, Verfahren zu ihrer Herstellung sowie ihre Verwendung als Arzneimittel, insbesondere gegen Diabetes, Hyperlipoproteinämie, Atherosklerose und Adipositas.

Es ist bereits bekannt geworden, dass N-Alkyl- und N-Alkenylderivate des 2-Hydroxymethyl-3,4,5-trihydroxypiperidins potente Inhibitoren für α-Glucosidhydrolasen sind. Darüber hinaus ist bekannt geworden, dass N-Alkylderivate dieser Verbindungen mit Alkylresten mittlerer Kettenlänge ($C_5$ bis $C_{14}$) die Lipidabsorption aus dem Darm hemmen (südafrikanische Patentanmeldung Nr. 78/4842). Die letztgenannten Verbindungen weisen aber eine gegenüber dem 1-Methyl-2-hydroxymethyl-3,4,5-trihydroxypiperidin abgeschwächte Wirkung auf die α-Glucosidhydrolasen auf.

Es wurden nun neue Derivate des 2-Hydroxymethyl-3,4,5-trihydroxypiperidins gefunden, die maximale Wirkung auf α-Glucosidhydrolasen und Lipidabsorption in sich vereinigen. Die neuen Verbindungen entsprechen der Formel I

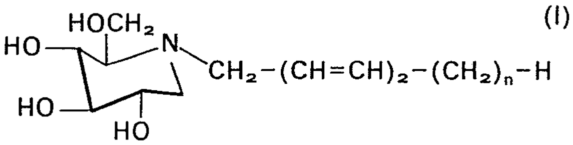

(I)

worin

n eine ganze Zahl zwischen 0 und 5 ist.

Die Verbindungen der vorliegenden Anmeldungen weisen gegenüber denen der südafrikanischen Patentanmeldung Nr. 78/4842 (entsprechend der EP-A1-947) den überraschenden Vorteil auf, gleichzeitig sehr wirksame α-Glucosidaseninhibitoren und sehr wirksame Inhibitoren der Fettabsorption aus dem Darm zu sein.

Verbindungen der Formel I erhält man, wenn man 2-Hydroxymethyl-3,4,5-trihydroxypiperidin-(1-desoxynojirimycin) in Gegenwart eines Wasserstoffdonors mit einem Aldehyd der Formel II

$$OCH-(CH=CH)_2-(CH_2)_n-H \quad (II)$$

in der

n die oben angegebene Bedeutung hat,

reduktiv alkyliert.

Die neuen Verbindungen erhält man weiterhin, wenn man 1-Desoxynojirimycin mit Alkylhalgeniden der Formel III

$$X-CH_2-(CH=CH)_2-(CH_2)_n-H \quad (III)$$

in der

n die oben angegebene Bedeutung hat, und

X ein Halogenatom, insbesondere Chlor, Brom oder Jod bedeutet,

zur Reaktion bringt.

Setzt man 1-Desoxynojirimycin mit Sorbindaldehyd und $NaCNBH_3$ als Wasserstoffdonor um, so lässt sich der Reaktionsablauf wie folgt formulieren:

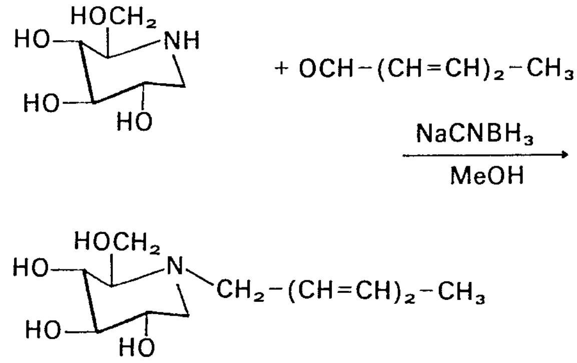


Mit 1-Desoxynojirimycin und 1-Bromhexadien-2,4 ergibt sich folgendes Reaktionsschema:

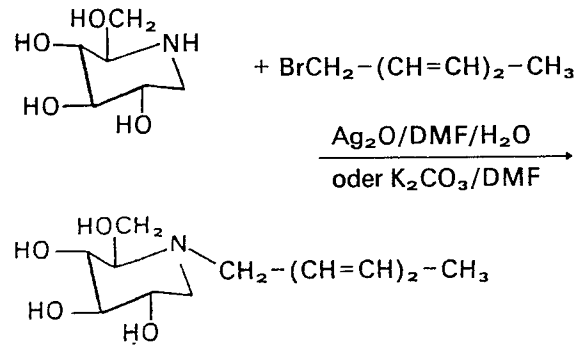


1-Desoxynojirimycin und die Aldehyde der Formel II [E.L. Pippen und M. Nonaka, „J. Org. Chem." 23, 1580 (1958)] bzw. Halogenide der Formel III [M. Jacobson, „J. Amer. Chem. Soc." 77, 2461 (1955)] sind literaturbekannt oder lassen sich nach literaturbekannten Verfahren herstellen.

Für die reduktive Alkylierung werden als Wasserstoffdonor-Reduktionsmittel Alkalimetallcyanoborhydride, Dialkylaminoborane und Alkalimetallborhydride verwendet. Besonders bevorzugt in dieser Verfahrensvariante ist die Verwendung von Natriumcyanoborhydrid. Die Reaktion wird im allgemeinen bei Temperaturen zwischen −20°C und Raumtemperatur durchgeführt. Es kann aber auch günstig sein, auf Rückflusstemperatur zu erhitzen.

Das Verfahren wird üblicherweise in einem inerten Lösungsmittel durchgeführt. Obwohl wasserfreie aprotische Lösungsmittel eingesetzt werden können (z.B. Tetrahydrofuran, wenn das Reduktionsmittel Morpholinoboran ist), wird gewöhnlich doch ein protisches Lösungsmittel verwendet. Als solches eignet sich besonders ein niederes Alkanol. Es kann aber auch Wasser oder ein wässeriges niedriges Alkanol (z.B. wässeriges Methanol oder Äthanol) oder andere wässerige Lösungsmittelsysteme, wie z.B. wässeriges Dimethylformamid, wässeriges Hexamethylphosphorsäuretriamid, wässeriges Tetrahydrofuran oder wässeriger Äthylenglykoldimethyläther verwendet werden.

Das Verfahren wird gewöhnlich in einem pH-Bereich von 1 bis 11 durchgeführt, bevorzugt ist ein pH-Bereich zwischen 4 und 7.

Die Umsetzung von 1-Desoxynojirimycin mit den Alkylhalogeniden wird in polaren, protischen oder aprotischen Lösungsmitteln zweckmässigerweise in Gegenwart eines säurebindenden Mittels bei Temperaturen zwischen 0° C und Siedetemperatur des Lösungsmittels durchgeführt.

Als säurebindende Mittel werden insbesondere Alkali- und Erdalkalihydroxide, -carbonate und -hydrogencarbonate, Silberoxid, Ammoniak und organische Amine wie Triäthylamin oder Pyridin eingesetzt.

Bevorzugt wird die Reaktion in Dimethylformamid/Wasser mit $Ag_2O$ als säurebindendem Mittel oder in Dimethylformamid mit Kaliumcarbonat als Säurefänger durchgeführt.

Als Wirkstoffe seien N-Hexadien-2,4-yl-1-desoxynojirimycin, N-Heptadien-2,4-yl-1-desoxynojirimycin, N-Octadien-2,4-yl-1-desoxynojirimycin, N-Nonadien-2,4-yl-1-desoxynojirimycin, N-Decadien-2,4-yl-1-desoxynojirimycin und N-Pentadien-z,4-yl-1-desoxynojirimycin genannt.

Es sei darauf hingewiesen, dass die meisten der genannten Wirkstoffe 4 stereoisomere Verbindungen bezüglich der Anordnung der Substituenten an den beiden Doppelbindungen umfassen. Die Erfindung betrifft sowohl die einzelnen Stereoisomeren als auch Gemische derselben.

Die erfindungsgemässen Inhibitoren eignen sich als Therapeutika für folgende Indikationen bei Mensch und Tier:

Prädiabetes, Gastritis, Obstipation, Karies, Infektionen des Gastrointestinaltraktes, Meteorismus, Flatulenz, Hypertension, und besonders Atherosklerose, Adipositas, Diabetes und Hyperlipoproteinämie.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, dass es sich um Kombinationen der erfindungsgemässen Inhibitoren untereinander oder um Kombinationen der erfindungsgemässen Inhibitoren mit bereits bekannten handelt.

Vorteilhaft sind in manchen Fällen auch Kombinationen der erfindungsgemässen Inhibitoren mit bekannten oralen Antidiabetika (β-cytotrope Sulfonylharnstoffderivate und/oder blutzuckerwirksame Biguanide) sowie mit blutlipid-senkenden Wirkstoffen wie z.B. Clofibrat, Nicotinsäure, Cholestyramin und anderen.

Die Verbindungen können ohne Verdünnung, z.B. als Pulver oder in einer Gelatinehülle oder in Kombination mit einem Trägerstoff in einer pharmazeutischen Zusammensetzung appliziert werden.

Pharmazeutische Zubereitungen können eine grössere oder kleinere Menge des Inhibitors enthalten, z.B. 0,1 bis 99,5%, in Kombination mit einem pharmazeutisch verträglichen nichttoxischen, inerten Trägerstoff, wobei der Trägerstoff eine oder mehrere feste, halbfeste oder flüssige Verdünnungsmittel, Füllstoffe und/oder nichttoxische, inerte und pharmazeutisch verträgliche Formulierungshilfsmittel enthalten kann. Solche pharmazeutischen Zubereitungen liegen vorzugsweise in Form von Dosierungseinheiten vor, d.h. physikalisch-diskreten, eine bestimmte Menge des Inhibitors enthaltenden Einheiten, die einem Bruchteil oder einem Vielfachen der Dosis entsprechen, die zur Herbeiführung der gewünschten Hemmwirkung erforderlich sind. Die Dosierungseinheiten können 1, 2, 3, 4 oder mehr Einzeldosen oder ½, ⅓ oder ¼ einer Einzeldosis enthalten. Eine Einzeldosis enthält vorzugsweise eine genügende Menge Wirkstoff, um bei einer Applikation gemäss eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen Haupt- und Nebenmahlzeiten am Tage verabreicht wird.

Andere therapeutische Mittel können auch eingenommen werden. Obgleich die Dosierung und das Dosierungsschema in jedem Fall sorgsam abgewogen werden sollte, unter Anwendung gründlichen fachmännischen Urteils und unter Beachtung des Alters, des Gewichts und des Zustands des Patienten, der Art und der Schwere der Erkrankung, wird die Dosierung gewöhnlich in einem Bereich zwischen etwa 0,5 bis etwa 100 mg/kg des Körpergewichtes pro Tag liegen. In manchen Fällen wird man dabei eine ausreichende therapeutische Wirkung mit einer geringeren Dosis erreichen, während in anderen Fällen eine grössere Dosis erforderlich sein wird.

Der europäischen Offenlegungsschrift Nr. 947 kann entnommen werden, in welcher Weise die erfindungsgemässen Verbindungen formuliert und appliziert werden.

*Saccharaseinhibitionstest* in vitro

Der Saccharaseinhibitionstest *in vitro* ermöglicht die Bestimmung der enzyminhibitorischen Aktivität einer Substanz durch den Vergleich der Aktivität des solubilisierten intestinalen Disaccharidasenkomplexes in Gegenwart bzw. in Abwesenheit (sog. 100-%-Wert) des Inhibitors. Als Substrat, welches die Spezifität des Inhibitionstestes bestimmt, dient dabei eine praktisch glucosefreie Saccharose (Glucose $<100$ ppm); die Enzymaktivitätsbestimmung basiert auf der spektrophotometrischen Bestimmung freigesetzter Glucose mittels Glucosedehydrogenase und Nicotinamidadenindinucleotid als Cofaktor.

Eine Saccharaseinhibitoreinheit (SIE) ist definiert als diejenige inhibitorische Aktivität, welche in einem definierten Testansatz eine vorgegebene saccharolytische Aktivität um eine Einheit (Saccharaseeinheit = SE) reduziert; die Saccharaseeinheit ist dabei als diejenige Enzymaktivität definiert, welche unter vorgegebenen Bedingungen 1 µmol Saccharose/min spaltet und damit zur Freisetzung von je 1 µmol Glucose, welche im Test bestimmt wird, und Fructose, welche im Test nicht erfasst wird, führt.

Der intestinale Disaccharidasenkomplex wird aus Schweinedünndarmmucosa durch tryptische Verdauung, Fällung aus 66% Äthanol bei −20° C, Aufnehmen des Präzipitates in 100 mmol/Phosphatpuffer, pH 7,0 und abschliessende Dialyse gegen denselben Puffer gewonnen.

10 µl einer Probelösung, die so angesetzt ist, dass die Extinktion des Testansatzes mindestens 10%, jedoch nicht mehr als 25% unter der des 100-%-Wertes liegt, werden mit 100 µl einer Verdünnung des intestinalen Disaccharidasenkomplexes in 0,1 mol Maleinatpuffer, pH 6,25, versetzt und für 10 min bei 37° C vorinkubiert. Die Verdünnung des Disaccharidasenkomplexes ist auf eine Aktivität von 0,1 Se/ml einzustellen.

Anschliessend wird die saccharolytische Reaktion durch Zugabe von 100 µl einer 0,4 mol Lösung von Saccharose (Serva 35579) in 0,1 mol Maleinatpuffer, pH 6,25 gestartet und nach einer Inkubationsdauer von 20 min bei 37° C durch die Zugabe von 1 ml Glucosedehydrogenasereagenz (1 Fläschchen Glucose/Dehydrogenase/Mutarotase-Gemisch lyophilisiert (Merck 14053) und 331,7 mg β-Nicotinamidadenindinucleotid (freie Säure, Boehringer Reinheitsgrad I) in 250 ml 0,5 mol Trispuffer, pH 7,6 gelöst) abgestoppt. Zum Nachweis der Glucose wird 30 min bei 37° C inkubiert und schliesslich bei 340 nm gegen einen Reagenzienblank (mit Enzym, jedoch ohne Saccharose) photometriert.

Die Berechnung der Hemmaktivität von Inhibitoren ist dadurch erschwert, dass schon geringfügige Änderungen im Testsystem, beispielsweise ein geringfügig von Bestimmung zu Bestimmung variierender 100-%-Wert, von nicht mehr zu vernachlässigendem Einfluss auf das Testergebnis sind. Man umgeht diese Schwierigkeiten, indem man bei jeder Bestimmung einen Standard mitlaufen lässt; als Standard dient ein Saccharaseinhibitor der Formel $C_{25}H_{43}O_{18}N$, welcher eine spezifische Hemmaktivität von 77 700 SIE/g aufweist und bei eingesetzten Mengen von 10 bis 20 ng im Test zu einer Hemmung von oben spezifizierter Grössenordnung führt. Bei Kenntnis der Differenz der Extinktionen bei 340 nm von 100-%-Wert und durch Standard gehemmten Ansatz lässt sich aus der Extinktionsdifferenz von 100-%-Wert und durch die Probelösung gehemmtem Ansatz unter Berücksichtigung der eingesetzten Menge an Inhibitor in bekannter Weise dessen spezifische Hemmaktivität errechnen, ausgedrückt in Saccharaseinhibitoreinheiten pro Gramm (SIE/g).

*Herstellungsbeispiele*

*Beispiel 1:*

N-Hexadien-2,4-yl-1-desoxynojirimcyin

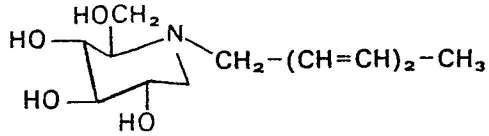

Eine Lösung von 5 g 1-Desoxynojirimycin in 100 ml Methanol und 4,5 ml Eisessig wird bei 0° C mit 4,4 ml Hexadienal und 3 g Natriumcyanoborhydrid versetzt. Nach einstündigem Rühren bei 0° C wird 18 h bei Raumtemperatur gerührt.

Es wird zur Trockne eingeengt, der Rückstand wird in Wasser aufgenommen und auf eine 120 cm lange und 3,5 cm weite Säule aufgetragen, die als stationäre Phase Cellulose und als mobile Phase Aceton enthält.

Zuerst wird mit Aceton, dann mit Aceton, dem schrittweise bis zu 30% Wasser zugefügt ist, eluiert. Die einzelnen Fraktionen werden dünnschichtchromatographisch überprüft, jene mit dem gewünschten Produkt zusammengefasst und eingeengt.

Man erhält nach Kristallisation mit Aceton 4 g der gesuchten Verbindung mit einem RF-Wert von 0,55 (Fliessmittel: Chloroform/Methanol/wässeriger Ammoniak 4:3:1), RF-Wert für 1-Desoxynojirimycin: 0,21, F.P.: 172-173° C.

*Beispiel 2:*

Analog Herstellungsbeispiel Nr. 1 wurde erhalten:

N-Heptadien-2,4-yl-1-desoxynojirimycin

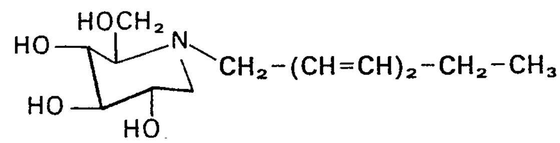

RF-Wert: 0,57, RF-Wert für 1-Desoxynojirimycin: 0,21, F.P.: 135-137° C.

*Beispiel 3:*

N-Hexadien-2,4-yl-1-desoxynojirimycin

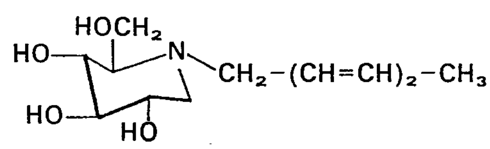

Eine Lösung von 25 g 1-Desoxynojirimycin in 500 ml Methanol und 22,5 ml Eisessig wird bei 0° C mit 22 ml Hexadienal und 15 g Natriumcyanoborhydrid versetzt. Nach einstündigem Rühren bei 0° C wird 18 h bei Raumtemperatur gerührt.

Anschliessend wird das Reaktionsgemisch auf eine mit Amberlite IR 120 ($H^{\oplus}$-Form) gefüllte Säule aufgetragen und die Säule zunächst mit Alkohol/Wasser 2:1 und dann mit Alkohol/Wasser 2:1, das 4% $NH_3$ enthält, eluiert. Das ammoniakalische Eluat wird zur Trockne eingeengt und der Rückstand aus Aceton kristallisiert. Das kristalline Produkt wird in Wasser gelöst und auf eine mit Amberlite IR 400 ($OH^{\ominus}$-Form) gefüllte Säule aufgetragen. Die Säule wird mit Alkohol/Wasser 1:1 eluiert und das Eluat am Rotationsverdampfer zur Trockne eingeengt. Der Rückstand wird aus wenig Wasser kristallisiert. Ausbeute: 14,4 g N-Hexadien-2,4-yl-1-desoxynojirimycin. F.P.: 172-173°C.

*Beispiel 4:*

Analog Beispiel 1 erhält man bei Verwendung von Pentadienal das N-Pentadien-2,4-yl-1-desoxynojirimycin.

*Beispiel 5:*

N-Hexa-2,4-dienyl-1-desoxynojirimycin

130,4 g 1-Desoxynojirimycin und 154,8 g fein gepulvertes $K_2CO_3$ werden in 1,3 l DMF gerührt. Dazu gibt man bei Raumtemperatur in einem Guss 180,3 g 1-Bromhexadien-2,4 in 200 ml DMF. Die Reaktionstemperatur steigt dabei auf ca. 40° C. Es wird weitere 2 h bei Raumtemperatur gerührt. Dann werden die Salze abgesaugt und der Nutschrückstand 2mal mit je 50 ml DMF nachgewaschen. Das Filtrat wird mit 2 l Eiswasser versetzt und in einem Scheidetrichter 2mal mit je 500 ml Diäthyläther extrahiert. Die Wasser/DMF-Phase wird am Rotationsverdampfer im Vakuum bei 50° C Badtemperatur zur Trockne gebracht. Der Rückstand wird mit 1,4 l Aceton verrührt und bei 0° C abgesaugt. Ausbeute: 150,5 g an salzhaltigem Rohprodukt. Das Rohprodukt wird aus 200 ml Wasser umkristallisiert. Ausbeute: 90 g. N-Hexadienyl-1-desoxynojirimycin vom F.P.: 165-168° C.

*Beispiel 6:*

Das nach Beispiel 3 erhaltene Produkt ist ein Gemisch zweier cis/trans isomerer Verbindungen. Es besteht nach HPLC und $H^1$-NMR-Untersuchungen zu 90-95% aus der trans/trans-Verbindung

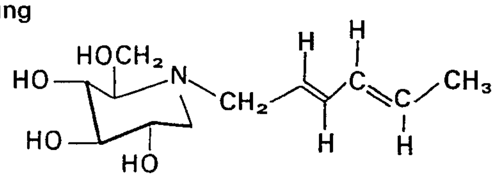

und zu 5-10% aus der trans/cis-Verbindung

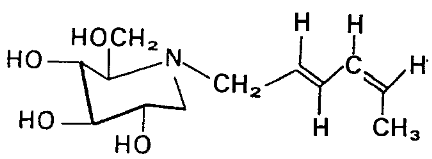

Durch Craig-Verteilung (Kreislaufverfahren) wurden die beiden Isomeren in dem System n-Butanol/Wasser 1:1 (Phasenverhältnis: 0,5) in 2325 Trennstufen getrennt. Aus 3,3 g Gemisch wurden 615 mg der trans/trans-Verbindung (Reinheit >99,5% nach HPLC) und 50 mg der trans/cis-Verbindung (Reinheit >98% nach HPLC; F.P.: 107-110° C) isoliert.

Beide Isomeren wurden *in vitro* auf saccharaseinhibierende Wirkung (Saccharaseinhibitiontest) geprüft. Während das trans/trans-Isomere die Saccharase aus Schweinedünndarmmucosa etwa 6mal stärker hemmt als 1-Desoxynojirimycin, ist das trans/cis-Isomere etwa 4mal wirksamer.

## Patentansprüche

1. Verbindung der Formel

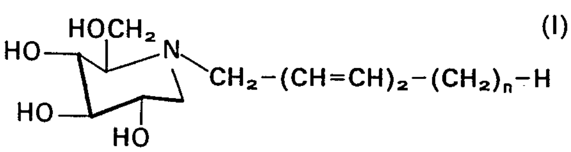

(I)

worin
n eine ganze Zahl zwischen 0 und 5 ist.

2. Verbindung gemäss Anspruch 1 der Formel

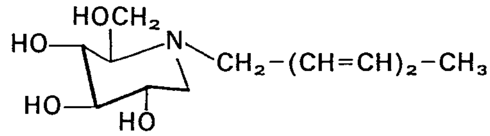

3. Verfahren zur Herstellung von Verbindungen gemäss Anspruch 1, dadurch gekennzeichnet, dass man 1-Desoxynojirimycin in Gegenwart eines Wasserstoffdonors mit einem Aldehyd der Formel

$$OCH-(CH=CH)_2-(CH_2)_n-H \quad (II)$$

worin
n eine ganze Zahl zwischen 0 und 5 ist,
reduktiv alkyliert.

4. Verfahren zur Herstellung von Verbindungen gemäss Anspruch 1, dadurch gekennzeichnet, dass man 1-Desoxynojirimycin mit Alkylhalogeniden der Formel

$$X-CH_2-(CH=CH)_2-(CH_2)_n-H \quad (III)$$

in der
n eine ganze Zahl zwischen 0 und 5 ist, und
X ein Halogen bedeutet,
zur Reaktion bringt.

5. Arzneimittel, gekennzeichnet durch einen Gehalt an einer Verbindung gemäss Anspruch 1 und gegebenenfalls pharmazeutisch geeigneten Zusatzstoffen.

6. Verbindungen gemäss Anspruch 1 zur Beeinflussung des Kohlenhydrat- und/oder Lipidstoffwechsels.

7. Verbindungen gemäss Anspruch 1 zur Behandlung von Adipositas, Diabetes, Hyperlipoproteinämie und Atherosklerose.

## Claims

1. Compounds of the formula:

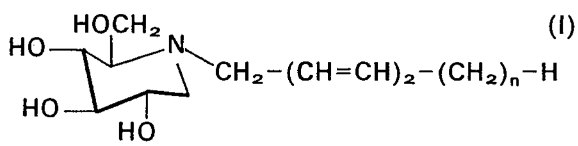

(I)

wherein n is an integer between 0 and 5.

2. Compound according to claim 1, of the formula:

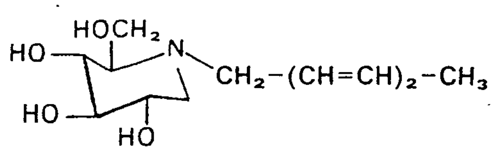

3. Process for the preparation of compounds according to claim 1, characterised in that 1-desoxynojirimicin is subjected to reductive alkylation with an aldehyde of the formula:

$$OCH-(CH=CH)_2-(CH_2)_n-H \quad (II)$$

wherein n is an integer between 0 and 5, in the presence of a hydrogen donor.

4. Process for the preparation of compounds according to claim 1, characterised in that 1-desoxynojirimicin is reacted with alkyl halides of the formula:

$$X-CH_2-(CH=CH)_2-(CH_2)_n-H \qquad (III)$$

in which n is an integer between 0 and 5 and X denotes a halogen.

5. Medicaments, characterised in that they contain a compound according to claim 1 and, if appropriate, pharmaceutically suitable additives.

6. Compounds according to claim 1 for influencing carbohydrate metabolism and/or lipid metabolism.

7. Compounds according to claim 1 for treating adiposity, diabetes, hyperlipoproteinaemia and atherosclerosis.

**Revendications**

1. Composés de formule (I):

(I)

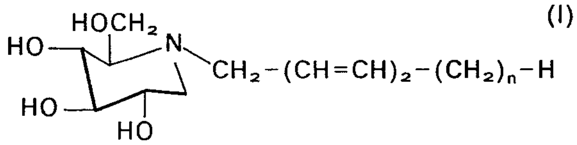

dans laquelle n est un nombre entier entre 0 et 5.

2. Composé selon la revendication 1, de formule:

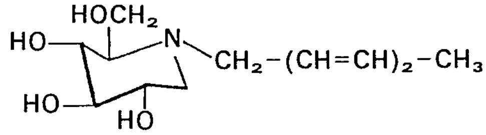

3. Procédé pour la fabrication des composés selon la revendication 1, caractérisé en ce que l'on effectue l'alkylation réductrice de la 2-hydroxyméthyl-3,4,5-trihydroxypipéridine-(1-désoxynojirimycine) avec un aldéhyde de formule (II);

$$OCH-(CH=CH)_2-(CH_2)_n-H \qquad (II)$$

dans laquelle n a la signification indiquée ci-dessus, en présence d'un donneur d'hydrogène.

4. Procédé pour la fabrication des composés selon la revendication 1, caractérisé en ce que l'on fait réagir la 1-désoxynojirimycine avec des halogénures d'alkyle de formule (III):

$$X-CH_2(CH=CH)_2-(CH_2)_n-H \qquad (III)$$

dans laquelle n a la signification indiquée ci-dessus et X représente un atome d'halogène.

5. Médicaments, caractérisés en ce qu'ils contiennent un composé selon la revendication 1 et éventuellement des additifs appropriés en pharmacie.

6. Composés selon la revendication 1, pour agir sur le métabolisme des hydrates de carbone et des lipides.

7. Composés selon la revendication 1, pour le traitement de l'adipose, du diabète, de l'hyperlipoprotéinémie et de l'athérosclérose.